# EUROPÄISCHE PATENTSCHRIFT

(11) **EP 3 278 694 B1**
(45) Veröffentlichungstag und Bekanntmachung des Hinweises auf die Patenterteilung: **14.10.2020**
(21) Anmeldenummer: 17184212.3
(22) Anmeldetag: 01.08.2017
(51) Int. Cl.: A47K 7/02

(54) **KONJAKSCHWAMM ZUM PEELING UND ZUR MASSAGE DER HAUT**
KONJAC SPONGE FOR PEELING AND MASSAGING THE SKIN
ÉPONGE EN KONJAC DESTINÉE AU PEELING ET AU MASSAGE DE LA PEAU

(30) Priorität: 02.08.2016 DE 102016214214
(43) Veröffentlichungstag der Anmeldung: 07.02.2018
(73) Patentinhaber: PARSA Haar- und Modeartikel GmbH, 74889 Sinsheim (DE)
(72) Erfinder: Hofer, Heiko, 68723 Oftersheim (DE)
(74) Vertreter: Altmann Stößel Dick Patentanwälte PartG mbB

(56) Entgegenhaltungen:
- EP-A1- 2 848 172
- CN-A- 102 167 846
- FR-A1- 2 939 669
- JP-A- 2003 250 717
- US-A1- 2016 206 522

## Beschreibung

### Gebiet der Erfindung

Die Erfindung betrifft einen Schwamm sowie ein Verfahren zur Herstellung eines Schwammes, wobei der Schwamm ein erstes Material enthaltend Glucomannane und ein zweites Material in Form von Partikeln umfasst, wobei die Partikel aus der zerkleinerten Walnussschalen bestehen. Vorzugsweise wird das erste Material erhalten oder ist erhältlich aus der Konjakwurzel. Insbesondere betrifft die Erfindung demnach einen Konjakschwamm sowie ein Verfahren zur Herstellung eines Konjakschwammes, wobei der Konjakschwamm die oben beschriebenen Partikel umfasst. Der Schwamm ist dabei insbesondere geeignet zur kosmetischen oder dermatologischen Behandlung der Haut, der Haare, der Schleimhaut und/oder der Hautanhangsgebilde, insbesondere zum Peeling und/oder Massage der Haut und/oder Hautanhangsgebilde.

Glucomannane (oder Glukomannane) sind stärkeähnliche Polysaccharide aus verzweigten Kohlenhydratketten. Die Hauptkette besteht dabei aus D-Mannose und D-Glucose, wobei in verschiedenen Abständen (kurze) Seitenketten auftreten. Sie kommen insbesondere in den Knollen von Amorphophallus Arten wie in beispielsweise *Amorphophallus* konjac, *Amorphophallus* kosiana, *Amorphophallus* shichitoensis, *Amorphophallus* companulatus, *Amorphophallus* variabilis bzw. *Amorphophallus* oncophyllus vor.

Insbesondere sind Glucomannane Hauptbestandteil der Knolle der Teufelszunge *Amorphophallus konjac,* auch Konjakwurzel genannt, aus der das Nahrungsmittel-Verdickungsmittel und das Sättigungsmittel Konjak hergestellt wird. Die Molekülmasse von Glucomannan aus Teufelszunge wird mit 200 bis 2000 kDa angegeben
Neben Nahrungsmitteln werden aus der Konjakwurzel auch Konjakschwämme hergestellt, die zur Behandlung der Haut verwendet werden.

Hier werden auch Konjakschwämme mit Zusätzen beschrieben, beispielsweise mit Aktivkohle (KR101075933) oder aber mit einem thermoreversiblen Gel (US 5,366,671). Das Dokument JP 2003 250717 A offenbart einen Schwamm, umfassend ein erstes Material enthaltend Glucomannane und ein zweites Material in Form von Partikeln. Diese Zusätze bewirken jedoch keine Verstärkung des Peeling-Effekts des Konjakschwammes und/oder eine Massage der Haut.

Es besteht demnach weiterhin ein Bedarf an Schwämmen, die zum verstärkten Peeling der Haut und insbesondere zur Massage der Haut geeignet sind.

### Aufgabe der Erfindung

Es ist daher Aufgabe der vorliegenden Erfindung, einen Schwamm und ein Verfahren zu dessen Herstellung bereitzustellen, mit welchem ein verstärkter Peeling Effekt und/oder eine vitalisierende Massage der Haut und/oder der Hautanhangsgebilde erreicht wird.

### Offenbarung der Erfindung

Diese Aufgabe wird gelöst durch einen Schwamm und ein Verfahren mit den Merkmalen der unabhängigen Patentansprüche. Vorteilhafte Weiterbildungen, welche einzeln oder in beliebiger Kombination realisierbar sind, sind in den abhängigen Ansprüchen dargestellt.

Insbesondere betrifft die Erfindung einen Schwamm, umfassend ein erstes Material enthaltend Glucomannane und ein zweites Material in Form von Partikeln, wobei die Partikel aus zerkleinerten Walnussschalen bestehen. Weiterhin betrifft die Erfindung ein Verfahren zur Erzeugung einer Konjakwurzelmasse M1 enthaltend Partikel, wobei die Partikel aus zerkleinerten Walnussschalen bestehen, umfassend die folgenden Schritte:
(i) Mahlen einer ersten Menge Konjakwurzel unter Erhalt eines Konjakwurzelpulvers
(ii) Hinzufügen mindestens einer Menge Partikel zum Konjakwurzelpulver aus (i) unter Erhalt eines Konjakwurzel-Partikel-Gemischs,
(iii) Hinzufügen mindestens einer vorbestimmten Menge Wasser zum Konjakwurzel-Partikel-Gemisch aus (ii)
unter Erhalt der Konjakwurzelmasse M1,
Weiterhin betrifft die Erfindung ein Verfahren zur Herstellung eines Schwamms umfassend
(a) Bereitstellung einer Konjakwurzelmasse M1 gemäß einem Verfahren wie oben beschrieben
(b) Erhitzen der Konjakwurzelmasse M1 aus (a), bevorzugt auf eine Temperatur im Bereich von 30 bis 120°C, unter Erhalt einer Konjakwurzelmasse M1*,
(c) Abkühlen der Konjakwurzelmasse M1* aus (b), bevorzugt auf eine Temperatur im Bereich von - 50 °C bis 25°C, unter Erhalt des Schwamms.

Weiterhin betrifft die Erfindung ein Verfahren zur Herstellung eines Schwamms umfassend mindestens zwei Schichten, wobei der Schwamm eine erste Schicht und eine zweite Schicht umfasst, wobei die erste Schicht aus mindestens 98 Gew.-% eines ersten Material besteht und wobei das erste Material erhalten wird oder erhältlich ist aus Konjakwurzel, und wobei die zweite Schicht das erste Material und ein zweites Material in Form von Partikeln enthält, wobei die Partikel aus zerkleinerten Walnussschalen bestehen, und wobei das Verfahren umfasst
(a) Bereitstellung einer Konjakwurzelmasse M1 gemäß einem Verfahren wie oben beschrieben,
(a2) Mahlen einer zweiten Menge Konjakwurzel unter Erhalt eines zweiten Konjakwurzelpulvers
(a3) Hinzufügen mindestens einer vorbestimmten Menge Wasser zum zweiten Konjakwurzelpulver aus (a2) unter Erhalt einer Konjakwurzelmasse M2
(a4) Bereitstellen einer Form, bevorzugt einer sterilisierten Form, und
   (a4)(1) Einfüllen der Konjakwurzelmasse M1 aus (a) in die Form,
   (a4) (2) Einfüllen der Konjakwurzelmasse M2 aus (a3) in die Form,
   wobei Schritt (a4)(1) vor oder nach Schritt (a4)(2) durchgeführt wird,
(b1) Erhitzen der Konjakwurzelmassen M1 und M2 aus (a4), bevorzugt auf eine Temperatur im Bereich von 30 bis 120 °C, unter Erhalt einer Konjakwurzelmasse M3
(c1) Abkühlen der Konjakwurzelmasse M3 aus (b1), bevorzugt auf eine Temperatur im Bereich von - 50 bis 20 °C,
unter Erhalt des Schwamms.

Weiterhin betrifft die Erfindung den Schwamm, herstellbar durch eines der oben geschriebenen Verfahren, sowie ein Kosmetikset, umfassend einen Schwamm wie oben beschrieben, oder einen Schwamm herstellbar nach einem der oben beschriebenen Verfahren, und einen luftundurchlässiges Behältnis, wobei der Schwamm in dem luftundurchlässigen Behältnis verpackt ist.

Weiterhin betrifft die Erfindung ein Verfahren zum Herstellen eines Kosmetiksets umfassend einen Schwamm wie oben beschreiben, oder einen Schwamm herstellbar nach einem der oben beschriebenen Verfahren, und ein luftundurchlässiges Behältnis, wobei der Schwamm in dem luftundurchlässigen Behältnis verpackt ist, das Verfahren umfassend die folgenden Schritte:
Herstellung eines Schwammes, wie oben beschrieben, und Einbringen des Schwamms in ein luftundurchlässiges Behältnis und Vakuumieren des luftundurchlässigen Behältnisses.

Im Folgenden werden die Begriffe "haben", "aufweisen", "umfassen" oder "einschließen" oder beliebige grammatikalische Abweichungen davon in nicht-ausschließlicher Weise verwendet. Dementsprechend können sich diese Begriffe sowohl auf Situationen beziehen, in welchen, neben den durch diese Begriffe eingeführten Merkmalen, keine weiteren Merkmale vorhanden sind, oder auf Situationen, in welchen ein oder mehrere weitere Merkmale vorhanden sind. Beispielsweise kann sich der Ausdruck "A hat B", "A weist B auf', "A umfasst B" oder "A schließt B ein" sowohl auf die Situation beziehen, in welcher, abgesehen von B, kein weiteres Element in A vorhanden ist (d.h. auf eine Situation, in welcher A ausschließlich aus B besteht), als auch auf die Situation, in welcher, zusätzlich zu B, ein oder mehrere weitere Elemente in A vorhanden sind, beispielsweise Element C, Elemente C und D oder sogar weitere Elemente.

Weiterhin wird darauf hingewiesen, dass die Begriffe "mindestens ein" und "ein oder mehrere" sowie grammatikalische Abwandlungen dieser Begriffe, wenn diese in Zusammenhang mit einem oder mehreren Elementen oder Merkmalen verwendet werden und ausdrücken sollen, das das Element oder Merkmal einfach oder mehrfach vorgesehen sein kann, in der Regel lediglich einmalig verwendet werden, beispielsweise bei der erstmaligen Einführung des Merkmals oder Elementes. Bei einer nachfolgenden erneuten Erwähnung des Merkmals oder Elementes wird der entsprechende Begriff "mindestens ein" oder "ein oder mehrere" in der Regel nicht mehr verwendet, ohne Einschränkung der Möglichkeit, dass das Merkmal oder Element einfach oder mehrfach vorgesehen sein kann.

Weiterhin werden im Folgenden die Begriffe "vorzugsweise", "insbesondere", "beispielsweise" oder ähnliche Begriffe in Verbindung mit optionalen Merkmalen verwendet, ohne dass alternative Ausführungsformen hierdurch beschränkt werden. So sind Merkmale, welche durch diese Begriffe eingeleitet werden, optionale Merkmale, und es ist nicht beabsichtigt, durch diese Merkmale den Schutzumfang der Ansprüche und insbesondere der unabhängigen Ansprüche einzuschränken. So kann die Erfindung, wie der Fachmann erkennen wird, auch unter Verwendung anderer Ausgestaltungen durchgeführt werden. In ähnlicher Weise werden Merkmale, welche durch "in einer Ausführungsform der Erfindung" oder durch "in einem Ausführungsbeispiel der Erfindung" eingeleitet werden, als optionale Merkmale verstanden, ohne dass hierdurch alternative Ausgestaltungen oder der Schutzumfang der unabhängigen Ansprüche eingeschränkt werden soll. Weiterhin sollen durch diese einleitenden Ausdrücke sämtliche Möglichkeiten, die hierdurch eingeleiteten Merkmale mit anderen Merkmalen zu kombinieren, seien es optionale oder nicht-optionale Merkmale, unangetastet bleiben.

### Partikel

Gemäß einer bevorzugten Ausführungsform enthält der Schwamm Partikel aus zerkleinerten Walnussschalen. Diese Partikel sind vorzugsweise so ausgebildet, dass sie einen geeigneten Peeling-Effekt und/oder eine geeignete Massage der Haut und/oder Hautanhangsgebilde, bevorzugt der Haut, ermöglichen. Es handlet sich bei den Partikeln um zerkleinerten Walnussschalen, bevorzugt um geschrotete oder gemahlene, bevorzugt geschrotete Walnussschalen.

Die Partikelgröße wird dabei bevorzugt so gewählt, dass die Partikel einen vorteilhaften Peeling-Effekt und/oder eine Massage der Haut und/oder Hautanhangsgebilde, bevorzugt der Haut, aufweisen. Bevorzugt weisen die Partikel eine Partikelgröße im Bereich von 100 bis 300 Mikrometer (100 bis 300 mesh), weiter bevorzugt im Bereich von 110 bis 200 Mikrometer, weiter bevorzugt im Bereich von 125 bis 180 Mikrometer, bestimmt gemäß Siebanalyse (mesh = Maschenweite des Siebs) auf.

Was die Menge des zweiten Materials innerhalb des Schwammes betrifft, so wird die Menge des zweiten Materials bevorzugt so gewählt, dass der gewünschte Peelingeffekt und/oder Massageeffekt erreicht wird. Bevorzugt enthält der Schwamm bzw. der Schwamm herstellbar oder hergestellt nach einem der hierin beschriebenen Verfahren, das zweite Material bevorzugt in einer Menge im Bereich 10 - 50 Gew.-%, weiter bevorzugt im Bereich von 15 - 40 Gew.-%, weiter bevorzugt im Bereich von 18 - 35 Gew.-%, weiter bevorzugt im Bereich von 19 - 33 Gew.-%, bezogen auf das Gesamtgewicht des Schwammes.

Bevorzugt weisen die Partikel eine Härte im Bereich von 2 bis 5 Mohs, weiter bevorzugt im Bereich von 2,5-3,5 Mohs auf.

### Schwamm-Material

Wie oben beschrieben, umfasst der Schwamm bzw. der Schwamm herstellbar oder hergestellt nach einem der hierin beschriebenen Verfahren, ein erstes Material enthaltend Glucomannane. Bevorzugt wird dieses erste Material aus der Konjakwurzel erhalten. Demnach betrifft die vorliegende Erfindung auch einen Schwamm, bzw. einen Schwamm herstellbar oder hergestellt nach einem der hierin beschriebenen Verfahren, wobei das erste Material erhalten wird oder erhältlich ist aus Konjakwurzel. Bevorzugt ist der Schwamm herstellbar oder wird hergestellt aus gemahlener Konjakwurzel.

Was die Menge des ersten Materials innerhalb des Schwammes betrifft, so enthält der Schwamm bzw. der Schwamm herstellbar oder hergestellt nach einem der hierin beschriebenen Verfahren, das erste Material bevorzugt in einer Menge im Bereich von 10 - 50 Gew.-%, weiter bevorzugt 15 - 40 Gew.-%, weiter bevorzugt 18 - 35 Gew.-%, weiter bevorzugt 19 - 33 Gew.-%, bezogen auf das Gesamtgewicht des Schwammes.

Bevorzugt sind mindestens 90 Gew.-% , bevorzugt mindestens 92 Gew.-% , weiter bevorzugt mindestens 96 Gew.-% , weiter bevorzugt mindestens 98 Gew.-% weiter bevorzugt 100% des ersten Materials, bezogen auf das Gesamtgewicht des ersten Materials, aus der Konjakwurzel erhalten oder erhältlich.

### Weitere Substanzen:

Der erfindungsgemäße Schwammes bzw. der Schwammes herstellbar oder hergestellt nach einem der hierin beschriebenen erfindungsgemäßen Verfahren, kann weitere Materialien enthalten. Zu nennen sind hier beispielsweise Wasser, Polymere, Tonerde und/oder beispielsweise oberflächenaktive Substanzen die, je nach Anwendungsgebiet als Tenside oder als Emulgatoren bezeichnet, aus anionischen, kationischen, zwitterionischen, ampholytischen und nichtionischen Tensiden und Emulgatoren ausgewählt sind. Ebenso kann der Schwamm bzw. der Schwamm herstellbar oder hergestellt nach einem der hierin beschriebenen erfindungsgemäßen Verfahren etherische Öle geringerer Flüchtigkeit, die meist als Aromakomponenten verwendet werden, und sich als Parfümöle eignen, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl, umfassen.

Bevorzugt enthält der Schwamm höchstens 4 Gew.-%, bevorzugt höchstens 1 Gew.-%, weiter bevorzugt höchstens 0,1 Gew.-%, weiter bevorzugt höchstens 0,01 Gew.-%, Wasser, bezogen auf das Gesamtgewicht des Schwamms.

Gemäß einer bevorzugten Ausführungsform enthält der erfindungsgemäße Schwamm bzw. der Schwamm herstellbar oder hergestellt nach einem der hierin beschriebenen erfindungsgemäßen Verfahren mindestens eine weitere Substanz ausgewählt aus der Gruppe bestehend aus grünem Tee, Kollagen, Aloe Vera, Bambuskohle, Kamille, Ingwer, Lavendel, Rose, Lycopin, Seide, Limone, Blaubeere, Perlenpulver und Aktivkohle. Enthält der Schwamm mindestens eine weitere Substanz, so enthält der Schwamm diese mindestens eine Substanz bevorzugt in einer Menge im Bereich von 0,5 bis 5 Gew.-% bezogen auf das Gesamtgewicht des Schwammes.

Gemäß einer alternativen bevorzugten Ausführungsform besteht der erfindungsgemäße Schwamm bzw. der Schwamm herstellbar oder hergestellt nach einem der hierin beschriebenen erfindungsgemäßen Verfahren im Wesentlichen aus dem ersten und dem zweiten Material. Im Wesentlichen bedeutet in diesem Zusammenhang, dass der Schwamm Reste von Wasser und anderen Substanzen, bevorzugt aber weniger als 0,1 Gew.-%, bezogen auf das Gesamtgewicht des Schwammes, enthalten kann.

Der Schwamm kann in beliebiger, optimal auf den jeweiligen Anwendungsbereich angepasster Form, bereitgestellt werden.

Gemäß einer bevorzugten Ausführungsform ist das zweite Material im Wesentlichen gleichmäßig im ersten Material verteilt.

Je nach gewünschten Anwendungsbereich, kann der erfindungsgemäße Schwamm bzw. der Schwamm herstellbar oder hergestellt nach einem der hierin beschriebenen erfindungsgemäßen Verfahren aus ein oder mehreren Schichten unterschiedlicher Zusammensetzung bestehen, wobei de Schwamm zusätzlich zu den ein oder mehreren Schichten eine Aufhängungsvorrichtung, bevorzugt eine schnurartige Vorrichtung, enthalten kann.

### Schwamm aus einer Schicht:

Gemäß einer bevorzugten Ausführungsform besteht der Schwamm aus einer Schicht und ggf. Aufhängungsvorrichtung, bevorzugt einer schnurartigen Vorrichtung. Weiter bevorzugt ist das zweite Material in dieser einen Schicht im Wesentlichen gleichmäßig im ersten Material verteilt. Gemäß dieser Ausführungsform ist bevorzugt die gesamte Schwammoberfläche (außer der optionalen Aufhängungsvorrichtung) zum Peeling und/oder zur Massage geeignet.

### Schwamm aus mindestens zwei Schichten

Gemäß einer weiteren bevorzugten Ausführungsform umfasst der erfindungsgemäße Schwamm bzw. der Schwamm herstellbar oder hergestellt nach einem der hierin beschriebenen erfindungsgemäßen Verfahren eine erste Schicht und eine zweite Schicht, und ggf. eine Aufhängungsvorrichtung, bevorzugt eine schnurartigen Vorrichtung, wobei die erste Schicht im Wesentlichen aus einem Teil des ersten Material besteht und, wobei die zweite Schicht das zweite Material und einen Teil des ersten Materials umfasst. Weiter bevorzugt ist in der zweiten Schicht das zweite Material im Wesentlichen gleichmäßig im ersten Material verteilt.

Bevorzugt entspricht die erste Schicht des Schwamms einer ersten Oberfläche des Schwamms und die zweite Schicht des Schwamms einer zweiten Oberfläche des Schwamms, wobei die zweite Oberfläche der ersten Oberfläche gegenüber liegt. Gemäß dieser Ausführungsform ist bevorzugt die zweite Schicht zum nicht-therapeutischen Peeling und/oder zur Massage der Haut und/oder der Hautanhangsgebilde, bevorzugt der Haut, geeignet.

Bevorzugt umfasst der erfindungsgemäße Schwamm bzw. der Schwamm herstellbar oder hergestellt nach einem der hierin beschriebenen erfindungsgemäßen Verfahren eine erste Schicht und eine zweite Schicht, wobei die erste Schicht aus mindestens 98 Gew.-% eines ersten Material besteht und wobei das erste Material erhalten wird oder erhältlich ist aus Konjakwurzel, und wobei die zweite Schicht das erste Material und ein zweites Material in Form von Partikeln enthält, wobei die Partikel aus zerkleinerten Walnussschalen bestehen. Bevorzugt enthält die erste Schicht keine Partikel aus zerkleinerten Walnussschalen.

### Verfahren:

Weiterhin betrifft die Erfindung auch ein Verfahren, wie oben beschrieben, zur Herstellung eines Schwamms umfassend
(a) Bereitstellung einer Konjakwurzelmasse M1 gemäß einem Verfahren wie oben beschrieben
(b) Erhitzen der Konjakwurzelmasse M1 aus (a), bevorzugt auf eine Temperatur im Bereich von 30 bis 120°C, unter Erhalt einer Konjakwurzelmasse M1*,
(c) Abkühlen der Konjakwurzelmasse M1* aus (b), bevorzugt auf eine Temperatur im Bereich von - 50 °C bis 25°C,
unter Erhalt des Schwamms.

Ebenso betrifft die Erfindung ein Verfahren zur Herstellung eines Schwamms umfassend mindestens zwei Schichten, wie oben beschrieben,
(a) Bereitstellung einer Konjakwurzelmasse M1 gemäß einem Verfahren nach wie oben beschrieben,
(a2) Mahlen einer zweiten Menge Konjakwurzel unter Erhalt eines zweiten Konjakwurzelpulvers
(a3) Hinzufügen mindestens einer vorbestimmten Menge Wasser zum zweiten Konjakwurzelpulver aus (a2) unter Erhalt einer Konjakwurzelmasse M2
(a4) Bereitstellen einer Form, bevorzugt einer sterilisierten Form, und
   (a4)(1) Einfüllen der Konjakwurzelmasse M1 aus (a) in die Form,
   (a4) (2) Einfüllen der Konjakwurzelmasse M2 aus (a3) in die Form,
   wobei Schritt (a4)(1) vor oder nach Schritt (a4)(2) durchgeführt wird,
(b1) Erhitzen der Konjakwurzelmassen M1 und M2 aus (a4), bevorzugt auf eine Temperatur im Bereich von 30 bis 120 °C, unter Erhalt einer Konjakwurzelmasse M3
(c1) Abkühlen der Konjakwurzelmasse M3 aus (b1), bevorzugt auf eine Temperatur im Bereich von -50 bis 20 °C,
unter Erhalt des Schwamms.

### Schritt (a)

Die Konjakwurzelmasse M1 wird bevorzugt durch ein Verfahren umfassend die folgenden Schritt bereitgestellt:
(i) Mahlen einer ersten Menge Konjakwurzel unter Erhalt eines Konjakwurzelpulvers
(ii) Hinzufügen mindestens einer Menge Partikel zum Konjakwurzelpulver aus (i) unter Erhalt eines Konjakwurzel-Partikel-Gemischs,
(iii) Hinzufügen mindestens einer vorbestimmten Menge Wasser zum Konjakwurzel-Partikel-Gemisch aus (ii)
unter Erhalt der Konjakwurzelmasse M1,

Das Mahlen der Konjakwurzel in Schritt (i) bzw. (a2) erfolgt nach dem Fachmann bekannten Verfahren.

### Schritt (b) und (b1)

In Schritt (b) bzw. (b1) wird die Masse M1 bzw. die Massen M1 und M2, bevorzugt auf eine Temperatur im Bereich von 30 bis 120°C, bevorzugt im Bereich von 50 bis 110 °C, weiter bevorzugt im Bereich von 80 bis 100 °C, erhitzt. Bevorzugt erfolgt diese Erhitzung in einem geeigneten Ofen.

Gemäß einer bevorzugten Ausführungsform erfolgt die Erhitzung gemäß (b) bzw. (b1) in einer Form, bevorzugt eine sterilisierte Form, wobei es sich bei der Form bevorzugt um eine Backform handelt.

Bevorzugt erfolgt das Erhitzen über einen Zeitraum von mindestens 5 Minuten.

### Schritt (c) bzw. (c1)

In Schritt (c) bzw. (c1) wird die Masse M1* bzw. M3, auf eine Temperatur im Bereich von -50 bis 25 °C, bevorzugt im Bereich von - 35 bis 0 °C abgekühlt. Bevorzugt erfolgt das Abkühlen über einen Zeitraum von mindestens 5 Minuten.

Die Schaummasse kann anschließend mit geeigneten, dem Fachmann bekannten Verfahren in verschiedene, für den jeweiligen Anwendungsbereich angepasste Formen gebracht werden, beispielsweise geschnitten oder gestanzt werden. Alternativ werden Formen, bevorzugt Backformen, mit der gewünschten Form verwendet. Wird eine Backform verwendet, so weist diese bevorzugt eine Halbkugelform, Tropfenform, Herzform oder länglich-flache Form auf.

Gemäß einer bevorzugten Ausführungsform umfasst das Verfahren weiterhin das Befestigen einer Aufhängungsvorrichtung, bevorzugt einer schnurartigen Vorrichtung, an dem abgekühlten Schwamm. Geeignete Vorrichtungen sind dem Fachmann bekannt.

### Verwendung des Schwamms

Der erfindungsgemäße Schwamm bzw. der Schwamm herstellbar oder hergestellt nach einem der hierin beschriebenen erfindungsgemäßen Verfahren ist bevorzugt zur kosmetischen oder dermatologischen Behandlung der Haut, der Haare, der Schleimhaut und/oder der Hautanhangsgebilde geeignet ist, insbesondere zur nicht-therapeutischen kosmetischen oder dermatologischen Behandlung der Haut, der Haare, der Schleimhaut und/oder der Hautanhangsgebilde. Insbesondere ist der Schwamm zum Peeling und/oder zur Massage der Haut und/oder der Hautanhangsgebilde, bevorzugt der Haut, geeignet.

### Kurze Beschreibung der Figuren

Weitere Einzelheiten und Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente.

Im Einzelnen zeigen:
- Figur 1: einen Schwamm 100 gemäß einer ersten Ausführungsform der vorliegenden Erfindung; und
- Figur 2: einen Schwamm 200 gemäß einer zweiten Ausführungsform der vorliegenden Erfindung.

### Beschreibung der Ausführungsbeispiele

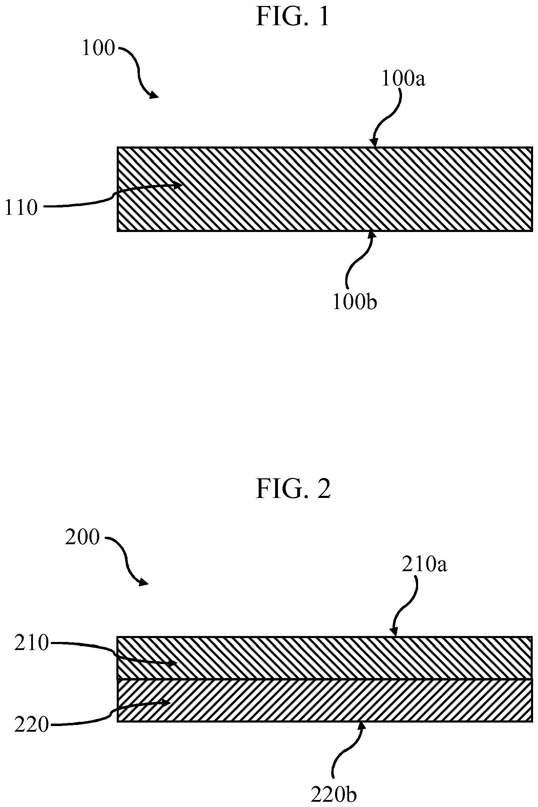

Figur 1 zeigt einen Schwamm 100 gemäß einer ersten Ausführungsform der vorliegenden Erfindung. Schwamm 100 weist eine Glucomannane-Partikel-Schicht 110 auf. Dabei umfasst Glucomannane-Partikel-Schicht 110 ein erstes Material enthaltend Glucomannane und ein zweites Material in Form von Partikeln. Das zweite Material in Glucomannane-Partikel-Schicht 110 ist im Wesentlichen gleichmäßig im ersten Material verteilt. Die Partikel bestehen aus zerkleinerten Walnussschalen.

Schwamm 100 ist zur kosmetischen oder dermatologischen Behandlung der Haut, der Haare, der Schleimhaut und/oder der Hautanhangsgebilde geeignet. Schwamm 100 ist insbesondere zur nicht-therapeutischen kosmetischen oder dermatologischen Behandlung der Haut, der Haare, der Schleimhaut und/oder der Hautanhangsgebilde geeignet. Insbesondere ist Schwamm 100 zum Peeling und/oder zur Massage der Haut und/oder der Hautanhangsgebilde geeignet.

Schwamm 100 weist ferner eine Schwammoberseite 100a und eine Schwammunterseite 100b auf. Schwammoberseite 100a liegt Schwammunterseite 100b gegenüber. Optional weist Schwamm 100 ferner eine schnurartige Vorrichtung (nicht gezeigt) auf. In der hier beschriebenen Ausführungsform ist die gesamte Schwammoberfläche (außer der schnurartigen Vorrichtung) zum Peeling und/oder zur Massage geeignet. Insbesondere sind Schwammoberseite 100a und Schwammunterseite 100b zum Peeling und/oder zur Massage geeignet.

Figur 2 zeigt einen Schwamm 200 gemäß einer zweiten Ausführungsform der vorliegenden Erfindung. Schwamm 200 weist eine Glucomannane-Partikel-Schicht 210 auf. Schwamm 200 weist ferner eine Glucomannaneschicht 220 auf. Dabei umfasst Glucomannane-Partikel-Schicht 210 ein erstes Material enthaltend Glucomannane und ein zweites Material in Form von Partikeln. Das zweite Material in Glucomannane-Partikel-Schicht 210 ist im Wesentlichen gleichmäßig im ersten Material verteilt. Die Partikel bestehen aus zerkleinerten Walnussschalen. Glucomannaneschicht 220 ist ein Beispiel für eine erste Schicht. Glucomannane-Partikel-Schicht 210 ist ein Beispiel für eine zweite Schicht.

Schwamm 200 ist zur kosmetischen oder dermatologischen Behandlung der Haut, der Haare, der Schleimhaut und/oder der Hautanhangsgebilde geeignet. Schwamm 200 ist insbesondere zur nicht-therapeutischen kosmetischen oder dermatologischen Behandlung der Haut, der Haare, der Schleimhaut und/oder der Hautanhangsgebilde geeignet. Insbesondere ist Schwamm 200 zum Peeling und/oder zur Massage der Haut und/oder der Hautanhangsgebilde geeignet.

Schwamm 200 weist ferner eine Schwammoberseite 210a und eine Schwammunterseite 220b auf. Schwammoberseite 210a liegt Schwammunterseite 220b gegenüber. Optional weist Schwamm 200 ferner eine schnurartige Vorrichtung (nicht gezeigt) auf. In der hier beschriebenen Ausführungsform ist Glucomannane-Partikel-Schicht 210 zum nicht-therapeutischen Peeling und/oder zur Massage der Haut und/oder der Hautanhangsgebilde geeignet.

### Ausführungsbeispiele

Die folgenden erfindungsgemäßen Schwämme wurden hergestellt und weisen die bevorzugten Peeling und Massage Eigenschaften auf:

| Type | Schwamm mit einer Schicht | | | | Schwamm mit zwei Schichten | | | |
|---|---|---|---|---|---|---|---|---|
| | Walnussschalen-Partikel | Konjakpulver | Gesamtgewicht Schwamm | Gew.-% Walnussschalen-Partikel | Walnussschalen-Partikel | Konjak pulver | Gesamt gewicht Schwamm | Gew.-% Walnussschalen-Partikel |
| Wasser sertropfen form | 2 gram | 4.2∼4.5g | 6.2-6.5 g | 32.25-30.76 | 1 gram | 3.5∼3. 8g | 4.5-4.8g | 22.22-20.83 |
| Eckige Form mit Welle | 5 gram | 11.2∼11. 8g | 16.2-16.8 g | 30.86-29.76 | 2.5 gram | 9.5∼10 .1 g | 12.0-12.6 g | 20.83-19.84 |

### Bezugszeichenliste

- 100: Schwamm
- 110: Glucomannane-Partikel-Schicht
- 100a: Schwammoberseite
- 100b: Schwammunterseite
- 200: Schwamm
- 210: Glucomannane-Partikel-Schicht
- 220: Glucomannaneschicht
- 210a: Schwammoberseite
- 220b: Schwammunterseite

## Patentansprüche

1. Schwamm (100, 200), umfassend ein erstes Material enthaltend Glucomannane und ein zweites Material in Form von Partikeln, wobei die Partikel aus zerkleinerten Walnussschalen bestehen.

2. Schwamm nach Anspruch 1, wobei das erste Material erhalten wird oder erhältlich ist aus Konjakwurzel.

3. Schwamm nach Anspruch 1 oder 2, wobei der Schwamm zur kosmetischen oder dermatologischen Behandlung der Haut, der Haare, der Schleimhaut und/oder der Hautanhangsgebilde geeignet ist.

4. Schwamm nach einem der vorstehenden Ansprüche, wobei die Partikel eine Partikelgröße im Bereich von 100 bis 300 Mikrometer aufweisen.

5. Schwamm nach einem der vorstehenden Ansprüche, wobei die Partikel eine Härte im Bereich von 2 bis 5 Mohs aufweisen.

6. Schwamm nach einem der vorstehenden Ansprüche, wobei der Schwamm das zweite Material in einer Menge im Bereich 10 - 50 Gew.-%, bezogen auf das Gesamtgewicht des Schwammes, umfasst.

7. Schwamm nach einem der vorstehenden Ansprüche, wobei der Schwamm aus einer Schicht (110) und optional einer Aufhängungsvorrichtung besteht.

8. Schwamm nach einem der Ansprüche 1 bis 6, wobei der Schwamm eine erste Schicht (220) und eine zweite Schicht (210) umfasst, wobei die erste Schicht im Wesentlichen aus einem Teil des ersten Materials besteht und, wobei die zweite Schicht das zweite Material und einen Teil des ersten Materials umfasst.

9. Kosmetikset, umfassend einen Schwamm nach einem der Ansprüche 1 bis 8 und ein luftundurchlässiges Behältnis, wobei der Schwamm in dem luftundurchlässigen Behältnis verpackt ist.

10. Verfahren zur Erzeugung einer Konjakwurzelmasse M1 enthaltend Partikel, wobei die Partikel aus zerkleinerten Walnussschalen bestehen,
umfassend die folgenden Schritte:
(i) Mahlen einer ersten Menge Konjakwurzel unter Erhalt eines Konjakwurzelpulvers
(ii) Hinzufügen mindestens einer Menge Partikel zum Konjakwurzelpulver aus (i) unter Erhalt eines Konjakwurzel-Partikel-Gemischs,
(iii) Hinzufügen einer vorbestimmten Menge Wasser zum Konjakwurzel-Partikel-Gemisch aus (ii)
unter Erhalt der Konjakwurzelmasse M1.

11. Verfahren zur Herstellung eines Schwamms (100, 200) umfassend
(a) Bereitstellung einer Konjakwurzelmasse M1 gemäß einem Verfahren nach Anspruch 10,
(b) Erhitzen der Konjakwurzelmasse M1 aus (a), bevorzugt auf eine Temperatur im Bereich von 30 bis 120°C, unter Erhalt einer Konjakwurzelmasse M1*,
(c) Abkühlen der Konjakwurzelmasse M1* aus (b), bevorzugt auf eine Temperatur im Bereich von - 50 °C bis 25 °C,
unter Erhalt des Schwamms.

12. Verfahren zur Herstellung eines Schwamms (200) umfassend mindestens zwei Schichten, wobei der Schwamm eine erste Schicht (220) und eine zweite Schicht (210) umfasst, wobei die erste Schicht aus mindestens 98 Gew.-% eines ersten Material besteht und wobei das erste Material erhalten wird oder erhältlich ist aus Konjakwurzel, und wobei die zweite Schicht das ersten Material und ein zweites Materials in Form von Partikeln umfasst, wobei die Partikel aus zerkleinerten Walnussschalen bestehen, wobei das Verfahren umfasst
(a) Bereitstellung einer Konjakwurzelmasse M1 gemäß einem Verfahren nach Anspruch 10,
(a2) Mahlen einer zweiten Menge Konjakwurzel unter Erhalt eines zweiten Konjakwurzelpulvers
(a3) Hinzufügen mindestens einer vorbestimmten Menge Wasser zum zweiten Konjakwurzelpulver aus (a2) unter Erhalt einer Konjakwurzelmasse M2
(a4) Bereitstellen einer Form, bevorzugt einer sterilisierten Form, und
(a4)(1) Einfüllen der Konjakwurzelmasse M1 aus (a) in die Form,
(a4) (2) Einfüllen der Konjakwurzelmasse M2 aus (a3) in die Form,
wobei Schritt (a4)(1) vor oder nach Schritt (a4)(2) durchgeführt wird,
(b1) Erhitzen der Konj akwurzelmassen M1 und M2 aus (a4), bevorzugt auf eine Temperatur im Bereich von 30 bis 120 °C, unter Erhalt einer Konjakwurzelmasse M3,
(c1) Abkühlen der Konjakwurzelmasse M3 aus (b1), bevorzugt auf eine Temperatur im Bereich von - 50 bis 20 °C,
unter Erhalt des Schwamms.

13. Schwamm nach einem der Ansprüche 1 bis 8 und erhalten oder erhältlich nach einem Verfahren nach Anspruch 11 oder 12.

14. Verfahren zum Herstellen eines Kosmetiksets nach Anspruch 9, umfassend die folgenden Schritte:
Durchführen des Verfahrens nach Anspruch 11 oder 12, und
Einbringen des Schwamms in ein luftundurchlässiges Behältnis und Vakuumieren des luftundurchlässigen Behältnisses.

## Claims

1. Sponge (100, 200) comprising a first material containing glucomannans and a second material in the form of particles, the particles consisting of comminuted walnut shells.

2. Sponge according to Claim 1, wherein the first material is obtained or is obtainable from konjac root.

3. Sponge according to Claim 1 or 2, wherein the sponge is suitable for the cosmetic or dermatological treatment of skin, of hair, of mucosa and/or of skin appendages.

4. Sponge according to any of the preceding claims, wherein the particles exhibit a particle size in the range from 100 to 300 micrometres.

5. Sponge according to any of the preceding claims, wherein the particles exhibit a hardness in the range from 2 to 5 Mohs.

6. Sponge according to any of the preceding claims, wherein the sponge comprises the second material in an amount in the range of 10-50% by weight, based on the total weight of the sponge.

7. Sponge according to any of the preceding claims, wherein the sponge consists of one layer (110) and optionally a hanging device.

8. Sponge according to any of Claims 1 to 6, wherein the sponge comprises a first layer (220) and a second layer (210), the first layer substantially consisting of a portion of the first material, and the second layer comprising the second material and a portion of the first material.

9. Cosmetics set comprising a sponge according to any of Claims 1 to 8 and an airtight container, with the sponge being packaged in the airtight container.

10. Process for generating a konjac root mass M1 containing particles, the particles consisting of comminuted walnut shells,
comprising the following steps:
(i) grinding a first amount of konjac root to obtain a konjac root powder
(ii) adding at least an amount of particles to the konjac root powder from (i) to obtain a konjac root/particles mixture,
(iii) adding a predetermined amounty of water to the konjac root/particles mixture from (ii) to obtain the konjac root mass M1.

11. Process for producing a sponge (100, 200), comprising
(a) providing a konjac root mass M1 by a process according to Claim 10,
(b) heating the konjac root mass M1 from (a), preferably to a temperature in the range from 30 to 120°C, to obtain a konjac root mass M1*,
(c) cooling the konjac root mass M1* from (b), preferably to a temperature in the range from -50°C to 25°C,
to obtain the sponge.

12. Process for producing a sponge (200) comprising at least two layers, the sponge comprising a first layer (220) and a second layer (210), the first layer consisting of at least 98% by weight of a first material and the first material being obtained or being obtainable from konjac root, and the second layer comprising the first material and a second material in the form of particles, the particles consisting of comminuted walnut shells, the process comprising
(a) providing a konjac root mass M1 by a process according to Claim 10,
(a2) grinding a second quantity of konjac root to obtain a second konjac root powder
(a3) adding at least one predetermined amount of water to the second konjac root powder from (a2) to obtain a konjac root mass M2
(a4) providing a mould, preferably a sterilized mould, and
(a4)(1) filling the konjac root mass M1 from (a) into the mould,
(a4)(2) filling the konjac root mass M2 from (a3) into the mould,
with step (a4)(1) being carried out before or after step (a4)(2),
(b1) heating the konjac root masses M1 and M2 from (a4), preferably to a temperature in the range from 30 to 120°C, to obtain a konjac root mass M3,
(c1) cooling the konjac root mass M3 from (b1), preferably to a temperature in the range from -50 to 20°C,
to obtain the sponge.

13. Sponge according to any of Claims 1 to 8 and obtained or obtainable by a process according to Claim 11 or 12.

14. Process for producing a cosmetics set according to Claim 9, comprising the following steps:
carrying out the process according to Claim 11 or 12, and
introducing the sponge into an airtight container and evacuating the airtight container.

## Revendications

1. Éponge (100, 200), comprenant un premier matériau contenant des glucomannanes et un deuxième matériau sous forme de particules, les particules étant constituées de coquilles broyées de noix.

2. Éponge selon la revendication 1, le premier matériau étant obtenu ou pouvant être obtenu à partir de racines de konjac.

3. Éponge selon la revendication 1 ou 2, l'éponge étant appropriée pour le traitement cosmétique ou dermatologique de la peau, des cheveux, des muqueuses et/ou des phanères.

4. Éponge selon l'une quelconque des revendications précédentes, les particules présentant une grosseur de particule dans la plage de 100 à 300 µm.

5. Éponge selon l'une quelconque des revendications précédentes, les particules présentant une dureté dans la plage de 2 à 5 Mohs.

6. Éponge selon l'une quelconque des revendications précédentes, l'éponge comprenant le deuxième matériau en une quantité dans la plage de 10-50% en poids, par rapport au poids total de l'éponge.

7. Éponge selon l'une quelconque des revendications précédentes, l'éponge étant constituée par une couche (110) et éventuellement par un dispositif de suspension.

8. Éponge selon l'une quelconque des revendications 1 à 6, l'éponge comprenant une première couche (220) et une deuxième couche (210), la première couche étant essentiellement constituée d'une partie du premier matériau et la deuxième couche comprenant le deuxième matériau et une partie du premier matériau.

9. Ensemble cosmétique, comprenant une éponge selon l'une quelconque des revendications 1 à 8 et un récipient imperméable à l'air, l'éponge étant emballée dans le récipient imperméable à l'air.

10. Procédé pour produire une masse de racine de konjac M1 contenant des particules, les particules étant constituées de coquilles broyées de noix, comprenant les étapes suivantes :
(i) broyage d'une première quantité de racine de konjac avec obtention d'une poudre de racine de konjac,
(ii) addition d'au moins une quantité de particules à la poudre de racine de konjac de (i) avec obtention d'un mélange racine de konjac-particules,
(iii) addition d'une quantité prédéfinie d'eau au mélange de racine de konjac-particules de (ii),
avec obtention de la masse de racine de konjac M1.

11. Procédé pour la fabrication d'une éponge (100, 200), comprenant
(a) la préparation d'une masse de racine konjac M1 selon un procédé selon la revendication 10,
(b) le chauffage de la masse de racine de konjac M1 de (a), de préférence à une température dans la plage de 30 à 120°C, avec obtention d'une masse de racine de konjac M1*,
(c) le refroidissement de la masse de racine de konjac M1* de (b), de préférence à une température de -50°C à 25°C,
avec obtention de l'éponge.

12. Procédé pour la fabrication d'une éponge (200) comprenant au moins deux couches, l'éponge comprenant une première couche (220) et une deuxième couche (210), la première couche étant constituée d'au moins 98% en poids d'un premier matériau et le premier matériau étant obtenu ou pouvant être obtenu à partir de racine de konjac et la deuxième couche comprenant le premier matériau et un deuxième matériau sous forme de particules, les particules étant constituées de coquilles broyées de noix, le procédé comprenant :
(a) la préparation d'une masse de racine konjac M1 selon un procédé selon la revendication 10,
(a2) le broyage d'une deuxième quantité de racine de konjac avec obtention d'une deuxième poudre de racine de konjac,
(a3) l'addition d'au moins une quantité prédéfinie d'eau à la deuxième poudre de racine de konjac de (a2) avec obtention d'une masse de racine de konjac M2
(a4) la mise à disposition d'un moule, de préférence d'un moule stérilisé, et
(a4) (1) l'introduction de la masse de racine de konjac M1 de (a) dans le moule,
(a4) (2) l'introduction de la masse de racine de konjac M2 de (a3) dans le moule,
l'étape (a4) (1) étant effectuée avant ou après l'étape (a4) (2),
(b1) le chauffage des masses de racine de konjac M1 et M2 de (a4), de préférence à une température dans la plage de 30 à 120°C, avec obtention d'une masse de racine de konjac M3,
(c1) le refroidissement de la masse de racine de konjac M3 de (b1), de préférence à une température de -50°C à 20°C,
avec obtention de l'éponge.

13. Éponge selon l'une quelconque des revendications 1 à 8 et obtenue ou pouvant être obtenue selon un procédé selon la revendication 11 ou 12.

14. Procédé pour la préparation d'un ensemble cosmétique selon la revendication 9, comprenant les étapes suivantes :
- mise en œuvre du procédé selon la revendication 11 ou 12 et
- introduction de l'éponge dans un récipient imperméable à l'air et mise sous vide du récipient imperméable à l'air.
